# EUROPEAN PATENT SPECIFICATION

(11) **EP 3 561 047 B1**
(45) Date of publication and mention of the grant of the patent: **06.01.2021**
(21) Application number: 19171463.3
(22) Date of filing: 26.04.2019
(51) Int. Cl.: C12N 1/06

(54) **PROCESS FOR THE PRODUCTION OF AN INACTIVE YEAST FOR USE IN OENOLOGY**
VERFAHREN ZUR HERSTELLUNG EINER INAKTIVEN HEFE ZUR VERWENDUNG IN DER ÖNOLOGIE
PROCÉDÉ DE PRODUCTION D'UNE LEVURE INACTIVE DESTINÉE À ÊTRE UTILISÉE EN OENOLOGIE

(30) Priority: 27.04.2018 IT 201800004962
(43) Date of publication of application: 30.10.2019
(73) Proprietor: ENOLOGICA VASON S.P.A., 37029 San Pietro in Cariano (VR) (IT)
(72) Inventor: VASON, Albano, 37029 San Pietro in Cariano (VR) (IT); GIOTTO, Federico, 31051 Follina (TV) (IT); ZANELLA, Gianmaria, 37015 Sant'Ambrogio di Valpolicella (VR) (IT)
(74) Representative: Gallo, Luca

(56) References cited:
- CN-A- 105 754 776
- GB-A- 538 191
- GB-A- 2 003 499
- US-A1- 2010 297 289
- HASHIZUME C ET AL: "KINETIC ANALYSIS OF YEAST INACTIVATION BY HIGH PRESSURE TREATMENT AT LOW TEMPERATURES", BIOSCI. BIOTECH. BIOCHEM,, vol. 59, no. 8, 1 August 1995 (1995-08-01) , pages 1455-1458, XP000530302, ISSN: 0916-8451
- OSMAN ERKMEN: "Mathematical modeling of Saccharomyces cerevisiae inactivation under high-pressure carbon dioxide", NAHRUNG - FOOD, vol. 47, no. 3, 1 June 2003 (2003-06-01), pages 176-180, XP055527301, DE ISSN: 0027-769X, DOI: 10.1002/food.200390041
- SENCER BUZRUL: "A REVIEW ON HIGH HYDROSTATIC PRESSURE TREATMENT OF WINE, GRAPE MUST AND GRAPE JUICE", CONFERENCE PAPER, 1 January 2012 (2012-01-01), XP055527190, DOI: 10.13140/RG.2.1.1386.6409
- Yang Tao ET AL: "High-Pressure Processing of Foods" In: "Emerging Technologies for Food Processing", 1 January 2014 (2014-01-01), Elsevier, NL, XP055527178, ISBN: 978-0-12-411479-1 pages 3-24, DOI: 10.1016/B978-0-12-411479-1.00001-2, * page 9, paragraph 1.3.1.3 *
- MARKO MALICANIN ET AL: "The effect of inactivated yeast-based products on the process of wine aging, phenolic compounds and sensory characteristics of red wine Prokupac", BIO WEB OF CONFERENCES, vol. 9, 1 January 2017 (2017-01-01), page 02004, XP055594726, DOI: 10.1051/bioconf/20170902004

## Description

### Field of application

The present invention regards a wine-making process and a process for producing an inactive yeast for use in wine-making according to the preamble of the respective independent claims 1 and 5.

The present process for producing an inactive yeast is intended for making a product advantageously employable in the enological/wine field for the treatment of enological liquids.

More in detail, the inactive yeast produced with the present method is advantageously employable for clarifying and/or for refining and treating musts and/or wines, or for improving the organoleptic characteristics and constituents of musts and wines as well as for determining lower oxidizability thereof.

The present processes therefore have optimal use in the wine-making field for the industrial production of wine.

### State of the art

Currently, as is known, the process of vinification of grapes provides for, after an initial step of crushing and pressing of the grapes, a step of alcoholic fermentation. In this step, there is the transformation of sugars into alcohol. The alcoholic fermentation of the musts can be obtained both through the natural microflora originally present in the grapes and through the introduction of specifically-selected yeasts belonging, as a function of the specific employed technique, to a single or to multiple yeast strains. Since the characteristics of the autochthonous yeasts naturally present in the musts depend on the environmental conditions and grape harvest technologies and on the wine cellar operations and situations, in order to improve and standardize the vinification processes it is known practice to add yeasts obtained from selected cultures. Generally, such yeasts are constituted by strains adapted for the specific vinifications; these are added in a quantity such to render them predominant with respect to the naturally present yeasts. It can also be provided to "deactivate" the natural microbial population, and introduce into the substrate one or more cultures that are also fully active.

The addition of controlled and specific microbial populations, generally in competition or substitution of the indigenous flora, allows obtaining numerous advantages including a faster course of the fermentation process, an increase of the ratio between ethyl alcohol content and sugar consumed, a reduction of the volatile acids, a quick clarification and a greater preservation capacity.

Alongside these primary activities relative to the actual fermentation process, the use of yeasts allows, especially if they are maintained present within the wine, also several further important "secondary" activities which above all regard the organoleptic characteristics of the wine and which therefore affect the sensory perceptions detectable when tasting. For example, it is in fact known that the presence of yeast can lead, through cell lysis phenomena, to the yielding of colloids capable of fixing aromatic substances, contributing to the structure of the wine and reducing irregular odors such as those due to hydrogen sulfide.

In addition, some substances derived from the yeast lysis stabilize the wine with regard to precipitations of potassium bitartrate and proteins.

Indeed, the enrichment with micro-molecules of wine due to the lysis or autolysis of the yeasts has a positive effect on the considered organoleptic characteristics. The mannoproteins released during the autolysis have the effect of preventing the crystallization of the tartaric acid salts, with an effectiveness which has proven comparable to that of a cold treatment.

Further positive effects deriving from the presence of yeast are due to the fact that the yeast being lysed frees enzymes which contribute to improving the evolution of the wines, to the fact that the yeast - even when dead - consumes dissolved oxygen and hence the wines preserved in the presence of yeast are less subject to oxidation phenomena and to the fact that the cell walls of the yeast can absorb various undesired substances, including fermentation catabolites, heavy metals, anti-parasite treatment residues or yellow-brownish pigments in white wines.

The aforesaid "secondary" actions are carried out by the yeasts both in the fermentation step and in a post-fermentation step in which the dead yeast or yeast being lysed (lees) is maintained within the wine: it can therefore be stated that the yeasts can be subjected - in the fermentation step and in a post-fermentation step - to a lysogenous action that determines a substantial improvement of the quality of the wines.

For such purpose, in many vinification processes it is provided to maintain the finest fraction of the lees in the wine after fermentation, such fraction mainly constituted by the yeast which produced the fermentation; in this step, repeated interventions are necessary for mixing the product in order to place the yeast back in suspension. However it is not always possible to intervene with such technology; indeed, frequently, reduction phenomena are verified which oblige immediate separation of the yeast from the wine, and thus the above-described positive "secondary" actions are not realized. Therefore, it is known that the presence of even only the lees of wines after fermentation has a positive effect on the characteristics of fineness and aromatic persistence, the refinement period on the lees requiring 6 to 8 months with periodic stirring operations (1-2 times per week) in order to facilitate the distribution of the yeast lees and facilitate the release of the parietal colloids during cell lysis.

The main drawback of this refinement process lies in the slowness with which the yielding of the polysaccharides by the lees occurs; this requires long maintenance times of the lees with the risk of irregular fermentations or the onset of reduction odor which would negatively affect the final quality of the wine.

In order to overcome this drawback, it is known to use enzymes with β-glucanase activity in order to accelerate the lysis process of the yeasts and obtain the desired effects on the wine already after several weeks of contact. Due to the use of β-glucanases, the enzymes facilitate an action of demolition of the cell wall of the yeasts, accelerating the release of the desired polysaccharide components.

Refinement processes are also known which provide for the use of inactive yeasts and glucanases in the refining step in order to obtain wines richer in polysaccharides, and with greater and more stable color.

In order to cause the lysis of the yeasts, in addition to the enzymatic technique described above, a deactivation process is known which provides for the use of ultrasounds for breaking the cell structures of the yeasts such that they release their protein fractions and speed up the kinetics of refinement of the wines on the fine lees. This technology has proven valid and comparable to the normal natural autolysis practices (refinement on the lees) and enzymatic lysis of the yeast, demonstrating that it leads to an increase of the total colloids, of the proteins and of the polysaccharides and a reduction of the diameter of the colloidal particles.

More recently, powders containing inactive yeasts have been introduced on the market that are capable of releasing mannoproteins and other substances that improve the refinement of the wine.

Such products comprise deactivated yeasts that derive from specific wine yeasts and differ from each other due to their composition, as they can comprise compounds which help improve the quality of the wine such as amino acids, peptides, polysaccharides, glutathione etc. (e.g. deactivated yeasts richer in glutathione can better protect against oxidation, improving the longevity of the aroma and color of the wine while deactivated yeasts richer in polysaccharides can better lead to a preservation of the color and give roundness to the wines). On this matter, it is also known from the patent FR 2926559 to make a product formed by deactivated dry yeasts enriched with glutathione.

These products are obtained by means of processes which provide for multiplication of the biomass, drying and a step of deactivation.

Therefore, in order to produce such deactivated yeast products, numerous deactivation techniques have been implemented, in particular aimed to determine the lysis of the yeasts in order to facilitate release of the substances useful for refining wine.

Among the different techniques employed for making the yeasts inert, there are chemical-physical processes such as acid, base or thermal treatments, which are employed separately or as an addition to enzymatic processes, and which are for example mentioned in the patent EP 1850682.

The processes employed up to now - ultrasound, thermal or chemical processes for deactivating the yeasts - have demonstrated that they are not fully satisfactory, since they do not allow completely preserving the protein chains, for example coming to break the tertiary or quaternary structure of the yeast proteins.

Pasteurization is also known as a method for deactivating yeasts. Nevertheless, like the abovementioned techniques, it leads the treated yeasts to high temperatures, once again deteriorating the structure of the yeast proteins.

The deactivation of contaminant microorganisms has been more recently studied, in particular in beer, by means of high-pressure homogenization (HPH). The required pressure - with normal pressurizing techniques - led to an increase of the temperature and consequently once again a damaging of the yeast proteins.

In addition, the products with deactivated yeast base available on the market are in dry powder state, and thus a state in which the original yeasts were dried, losing at least part of the integrity of their proteins such that once inserted in wine for refinement purpose they have an action that is not fully satisfactory.

As is known in nature, a great number of yeast species are available, yet those for wine-making use are few in number. Among the most common yeasts, as a non-limiting example there are the following: Saccharomyces cerevisiae, Saccharomyces uvarum, Saccharomyces chevalieri, Saccharomyces bayanus, Kloeckera apiculata, Pichia, etcetera. Quite often in wine-making, active dry yeasts are used, regarding which it is intended (as is known) yeasts selected for wine-making use, placed on the market in dry form or with a limited amount of water. Generally, these are preserved in the dry state for not over two years time. For their use, it is necessary to conduct a preliminary rehydration step. The effectiveness and the quality of their principal activity tied to the fermentation of the must is comparable to that of the yeasts prepared in suspension, and certainly better to that due to spontaneous fermentations. Nevertheless, during drying there is a deactivation of the enzymatic activity, and even after rehydration such activity results lower than that of the yeasts not subjected to the aforesaid drying, preservation and rehydration steps.

The non-optimal characteristics of the dry powder yeasts remain as is or they are worsened following the above-described deactivation phenomena which further damage the protein chains of the yeasts.

Known from the patent EP 1236795 is the use of active dough yeast for wine-making use (LPA), i.e. a yeast that was never subjected to drying or lyophilization and which has a water residue approximately around 60%. Nevertheless, an effective process has not yet been discovered for deactivating such dough yeasts that does not provide for the drying thereof and/or for subjecting them to the above-described deactivation techniques.

Therefore, up to now, there is the need to have a process for deactivating yeasts that allows providing yeast lysates that maintain intact the characteristics of their proteins in order to maximize the effectiveness of refinement in wine.

In the food field, it is known to achieve a cold pasteurization for destroying the microorganisms contained in the foods, by subjecting the foods themselves to extremely high pressures capable of breaking the biological structures of the same microorganisms. Several examples of such processes and apparatuses for the cold pasteurization are for example described in the patents US 2014010932 and US 20080050507.

At this regard it is know for instance from the article "The effect of inactivated yeast-based products on the process of wine aging, phenolic compounds and sensory characteristics of red wine Prokupac" BIO WEB OF CONFERENCES, vol. 9, 1 January 2017, page 02004 a method to inactive the yeast in order to release into the wine many bioactive compounds such as peptides, amino acids, mannoproteins etc (see). More in detail this document discloses a wine-making process which comprises the steps of arranging an inactive yeast for wine-making use and of adding, to an enological product, said inactive yeast such that it does not perform a fermentation action in said process.

In addition, there is the particular need to obtain a wine-making process capable of fully exploiting the mannoproteins and the colloidal fraction contained in the yeast lysates for the purpose of maximizing the performances thereof during clarification and/or refinement and treatment of musts and/or wines.

It is also known from the article "Kinetic analysis of yeast inactivation by high pressure treatment at low temperatures" BIOSCI. BIOTECH. BIOCHEM., vol. 59, no. 8, 1 August 1995, pages 1455-1458, a high-pressure (between 150 and 300 MPa) processing suitable to inactivate the yeast and that has been successfully applied to many processed foods and some of them such as jam and jelly are available on the market.

Also the article "Mathematical modeling of Saccharomyces cerevisiae inactivation under high-pressure carbon dioxide" NAHRUNG - FOOD, vol. 47, no. 3, 1 June 2003, pages 176-182 discloses a yeast inactivation method that provides to insert the yeast, within a pressurization chamber, a fluid at a pressure comprised between 25 and 100 bar in order to inactivate the yeast.

The Article "A review on high hydrostatic pressure treatment of wine, grape must and grape juice" CONFERENCE PAPER, 1 January 2012" disclose a method to the inactivation of bacteria and microorganisms in wine, grape must and grape juice by subjecting the food product as a whole to a high pressure HHP treatment.

### Presentation of the invention

The main object of the present invention is therefore that of overcoming the drawbacks shown by the abovementioned solutions of known type by providing a wine-making process capable of fully exploiting the components contained within yeast lysates, for the purpose of maximizing their performances during clarification and/or refinement and treatment of musts and/or wines.

A further object of the present invention is to provide a process for producing an inactive yeast for use in wine-making which is capable of providing a lysate, or a yeast derivative, in which the components contained within the cell wall of the original yeast are immediately available and ready to act.

A further object of the present invention is to provide a wine-making process and a process for producing an inactive yeast employable in wine-making, which allow improving the refinement of wines of different types: red, white and rose.

A further object of the present invention is to provide a process for producing an inactive yeast and a wine-making process employing such inactive yeast, capable of reducing the time employed for refining the wine.

A further object of the present invention is to provide a wine-making process and a process for producing an inactive yeast employable in wine-making, which allow improving the clarification of musts and wines.

A further object of the present invention is to provide a process for producing an inactive yeast which is easy to achieve.

A further object of the present invention is to provide a process for producing an inactive yeast for use in wine-making, in particular for the steps of clarification and refinement of musts and wines, which is compliant with laws in the wine-making field and is entirely reliable.

### Brief description of the drawings

The technical characteristics of the present invention, according to the aforesaid objects, can be seen in the contents of the below-reported claims and the advantages thereof will be more evident in the following detailed description, made with reference to the experimental graphs of the product according to the invention, in which:
- Fig. 1 shows a graph relative to an analysis of the color stabilization effect carried out on the wine by an inactive yeast produced with the process, object of the present invention;
- Fig. 2 shows a graph relative to an analysis of the protein stabilization effect carried out on the wine by an inactive yeast produced with the process, object of the present invention;
- Figs. 3A and 3B respectively show a graph relative to an analysis of the reduction effect of copper and of iron carried out on the wine by an inactive yeast produced with the process, object of the present invention;
- Figs. 4A, 4B and 4C show three graphs relative to an analysis of the clarification effect carried out on musts by an inactive yeast produced with the process, object of the present invention;
- Figs. 5A, 5B and 5C show three graphs relative to an analysis of the clarification effect on the components relative to the color of the wine by an inactive yeast produced with the process, object of the present invention;
- Figs. 6A and 6B show two graphs relative to an analysis of the oxidation protection effect carried out on the wine by an inactive yeast produced with the process, object of the present invention;
- Figs. 7A and 7B show two graphs relative to an analysis on the refinement times reduction effect carried out on the wine by an active yeast of known type and by an inactive yeast produced with the process, object of the present invention;
- Fig. 8 shows a photograph taken of three wine samples after an analysis of the tartaric stabilization effect carried out by an active yeast of known type and by an inactive yeast produced with the process, object of the present invention;
- Fig. 9 shows a graph relative to an analysis of the color stabilization effect carried out on the wine by an inactive yeast produced with the process, object of the present invention, and by an inactive yeast produced with a process of known type;
- Figs. 10 and 11 show two graphs relative to analysis of the protein stabilization effect carried out on two different wines by an inactive yeast produced with the process, object of the present invention, and by an inactive yeast produced with a process of known type;
- Figs. 12A-12B-12C, 13A-13B-13C and 14A-14B-14C show graphs relative to analyses of the clarification effect carried out on three different musts by an inactive yeast produced with the process, object of the present invention, and by an inactive yeast produced with a process of known type;
- Fig. 15 shows a graph relative to an analysis of the protective effect against the oxidation carried out on a must by an inactive yeast produced with the process, object of the present invention, and by an inactive yeast produced with a process of known type, in which the absorbance was measured of samples of musts treated with the different inactive yeasts;
- Figs. 16A, 16B and 16C show three graphs relative to an analysis of the protective effect against the oxidation carried out on the must of figure 15 by an inactive yeast produced with the process, object of the present invention, and by an inactive yeast produced with a process of known type, in which the color was analyzed of samples of musts treated with the different inactive yeasts;
- Figs. 17A-17B-17C and 18A-18B-18C show graphs relative to an analysis of the clarification effect on the components relative to the color of two different white wines by an inactive yeast produced with the process, object of the present invention, and by an inactive yeast produced with a process of known type;
- Figs. 19A-19B-19C, 20A-20B-20C and 21A-21B-21C show graphs relative to an analysis of the color of three different white wines after a treatment executed by means of the addition of an inactive yeast produced with the process, object of the present invention, and by means of the addition of an inactive yeast produced with a process of known type, in which the values were measured thirty days after the treatment;
- Figs. 22A-22B, 23A-23B, 24A-24B and 25A-25B show graphs relative to an analysis of the oxidation protection effect carried out on four different white wines by an inactive yeast produced with the process, object of the present invention, and by an inactive yeast produced with a process of known type;
- Figs. 26A and 26B respectively show a graph relative to an analysis of the reduction effect of the copper and iron carried out on the wine by an inactive yeast produced with the process, object of the present invention, and by an inactive yeast produced with a process of known type;
- Fig. 27 shows a graph relative to an analysis of the content of total colloids in an inactive yeast produced with a process of known type and in two inactive yeasts produced with the process, object of the present invention, and subsequently dried with two different techniques;
- Fig. 28 shows a graph relative to an analysis of the content of total thiol groups in an inactive yeast produced with a process of known type and in two inactive yeasts produced with the process, object of the present invention, and subsequently dried with two different techniques.

### Detailed description of a preferred embodiment of the present invention

In an entirely general manner, the vinification process comprises numerous treatment steps for treating first the must and then the wine in order to obtain a drink with suitable organoleptic characteristics. It substantially starts with the harvest of the grapes during grape harvest and is concluded with the maturation of the wine in suitable containers (tanks, casks and bottles) for the refinement of the qualities thereof. Immediately after the harvest, the grapes are subjected to a pressing step for producing the must which, as is known, by means of a main dose of autochthonous or inoculated yeasts undergoes the fermentation phenomenon.

It is in fact known that yeasts, due to their fermentation metabolism, after a first cell proliferation step, metabolize the sugars contained in the must, transforming them into ethyl alcohol and carbon dioxide, thus transforming the must into wine.

The process for producing an inactive yeast object of the present invention provides for obtaining an inactive yeast, i.e. more precisely a yeast derivative, which is not intended to perform a fermentation action and/or reproductive action for an enological liquid, but rather it is intended to perform an antioxidant action and/or stabilizing action for the enological liquid to which it is susceptible of being added, as is better explained hereinbelow.

Advantageously, such yeast to be added to the enological liquid is an inactive dough or cream yeast, which is susceptible of being added as is or dried (e.g. by means of techniques well-known in the wine-making field, such as cold lyophilization or spray dry) and is unsuitable to carry out fermentation activity, in which such inactive condition is reached by means of the process according to the present invention.

More in detail, in such document, with the term "cream yeast", otherwise termed Cream Yeast (CRY), it is intended a yeast with a content of dry matter preferably comprised between 18 and 25% and a level of vital yeasts equal to or greater than 10¹⁰ CFU/g of dry matter. In addition, in this document, with the term "dough yeast", otherwise termed Compressed Yeast (COY), it is intended a yeast with a dry matter content preferably comprised between 30 and 35% and a level of vital yeasts equal to or greater than 10¹⁰ CFU/g of dry matter. Advantageously, such definitions of cream yeast and dough yeast are complaint with that indicated by the OIV Resolution (International Organization of Vine and Wine) - OENO 576A -2017.

The process according to the present invention provides for a step of providing an active yeast for wine-making use, which is not dried and which contains a water residue of at least 40% by mass. Preferably, the active yeast to be provided is a cream yeast with water content around 75% by mass, or it is a dough yeast with a water content around 55% by mass, and preferably it is a dough yeast of the type described in the European patent EP 1236795, enclosed herein for reference purposes from paragraph [0036] to paragraph [0076].

In accordance with a further embodiment, the active yeast to be provided is a frozen yeast, otherwise termed Frozen Yeast (AFY). More in detail, in such document, with the term "frozen yeast", it is intended a yeast with a dry matter content preferably comprised between 40 and 85% and a level of vital yeasts equal to or greater than 10¹⁰ CFU/g of dry matter, as indicated by the Resolution OIV - OENO 576A -2017.

The present process then provides for the insertion of the active yeast, preferably in the above-described forms, in a pressurization chamber which substantially acts as hydrostatic autoclave.

There is then the insertion, within the pressurization chamber, of a fluid at a lysis pressure greater than 1000 bar in order to subject the active yeast to such lysis pressure. During such step, the compression of the cells of the active yeast takes place with consequent breakage of the molecular structure of the yeast itself, which leads to the lysis of the active yeast and to the obtainment of an inactive yeast.

By inactive yeast it must be intended an inactive yeast lysate comprising at least one colloidal fraction and mannoproteins, which are free and susceptible of reacting in wine or in must, in particular with non-fermentation effects.

Advantageously, such compression does not involve an increase of temperature of the yeast within the pressurization chamber if not of at most a few degrees (and in any case without ever reaching temperatures greater than 30° degrees) and hence does not determine any deterioration of the proteins of the yeast due to the heat. In addition, the use of a cream yeast or dough yeast, which has never been dried, allows further obtaining an inactive yeast which has preserved the integrity of the proteins contained within the cell wall. Indeed, experimentally, it was surprisingly observed that - once dried - the yeast no longer reacquires the characteristics of a yeast that had never been dried, regarding the non-fermentation actions.

The yeasts deactivated by means of pressure, in particular by means of pressures greater than 1000 bar, allow providing the colloidal fraction and the mannoproteins contained in the cell walls of the yeast, which preserve the characteristics of the starting active yeast and allow obtaining higher performance effects on the wine or on the must to which the inactive yeast is intended to be added, as was experimentally observed in the comparative analyses described hereinbelow.

In fact, the colloidal fractions and the mannoproteins of such yeasts deactivated by means of pressure are reasonably provided with longer protein chains with respect to the chains provided by other inactive yeasts and experimentally it was observed that these react more with the wine and the must to which they are added, producing higher-performing effects of clarification and stabilization.

There is then a step for depressurizing the aforesaid pressurization chamber together with a step for extracting the inactive yeast from the same pressurization chamber.

According to a first possible embodiment, the process for producing inactive yeast, object of the present invention, provides for advantageously inserting the active yeast, in dough or cream form, in the pressurization chamber of an apparatus for the discontinuous cold pasteurization. As is known, the apparatuses for discontinuous cold pasteurization provide for inserting a liquid in the pressurization chamber, generally water and preferably pure water, which allows reaching lysis pressures comprised in an interval between 1000 and 6500 bar.

Advantageously, in such first embodiment, the process provides for, before the insertion of the active yeast in the pressurization chamber, the insertion of the active yeast in at least one package at least partially made of flexible material, which is advantageously sealed, e.g. by means of a heat sealing, so as to prevent the pressurized water within the pressurization chamber from entering into the package, being mixed with the active yeast contained therein.

In operation, the material with which the package is attained must be capable of sustaining the compression imparted by the apparatus for the discontinuous pasteurization, without compromising the integrity of the package and/or of the sealing. Consequently, the package can advantageously be made of plastic material for foods, such as PET (polyethylene terephthalate), HDPE (high-density polyethylene), LDPE (low-density polyethylene) and PP (polypropylene); or it can be made of flexible aluminum, or of Tetrapak. In addition, the package can be made in bag, can or box form, or it can be made in the form of a rigid or semirigid tray sealed with a flexible cover material, and it can comprise a single layer or multiple layers of flexible material, or multiple layers sealed to each other.

Advantageously, moreover, before the sealing, the package can be subjected to a vacuum-forming step. In this manner, all of the active yeast contained in the package is advantageously subjected to the same lysis pressure since, within the package, there is no gas that is differently-compressible with respect to the active yeast itself.

The sealed package of active yeast is then inserted in the pressurization chamber and it is maintained at the lysis pressure for a time preferably comprised between 30 and 600 seconds.

In accordance with a second possible embodiment, the process for producing inactive yeast, object of the present invention, advantageously provides for inserting the active yeast, in dough or cream form, in the pressurization chamber of an apparatus for continuous cold pasteurization. As is known, the apparatuses for continuous cold pasteurization provide for inserting, in the pressurization chamber, a gas that is generally carbon dioxide, which allows reaching lysis pressure that are lower than the lysis pressures reached by the apparatuses for discontinuous cold pasteurization. More in detail, such apparatuses for continuous cold pasteurization allow obtaining a lysis pressure comprised in an interval between 1000 and 1500 bar.

Advantageously, in such second embodiment, the process does not require packaging the active yeast before inserting it into the pressurization chamber, since the carbon dioxide is not miscible with the yeast and hence it does not dilute the concentration thereof. In other words, in such second embodiment, the active yeast is advantageously directly inserted in the pressurization chamber of the apparatus for continuous cold pasteurization, without a package.

Advantageously, in this manner, the carbon dioxide inserted in the pressurization chamber of the apparatus for continuous cold pasteurization enters directly in contact with the yeast and produces at least one partial breakage of the cell membrane thereof. Indeed, the carbon dioxide has a corrosive effect on the cell membrane of the yeast and produces an at least partial lysis, and hence a lower lysis pressure is sufficient for obtaining an inactive yeast with characteristics comparable to the inactive yeast obtained by means of apparatuses for discontinuous cold pasteurization.

Preferably, the active yeast inserted in the pressurization chamber is then maintained at the lysis pressure for a time comprised between 30 and 600 seconds.

Advantageously, the process for producing inactive yeast, object of the present invention, can be made by means of the first or the second embodiment depending on the type of active yeast provided, depending on the desired lysis degree and on the type of treatment that the resulting inactive yeast will be susceptible of carrying out on an enological liquid. Indeed, the discontinuous cold pasteurization allows obtaining a more complete lysis of the active yeast with respect to the discontinuous cold pasteurization. More clearly, the inactive yeast can be employed in a satisfactory manner in the treatment of an already fermented enological liquid (e.g. for obtaining a wine refinement or clarification effect), also with a partial lysis of the yeast molecules obtained by means of reduced lysis pressure, since the lysis of the cell membrane also continues naturally once the inactive yeast is in contact with the fermented enological liquid.

In this case, the present process can advantageously provide for employing an apparatus for continuous cold pasteurization, or an apparatus for discontinuous cold pasteurization with limited lysis pressures, for example of about 3000 bar.

Otherwise, if the inactive yeast is to be used in treating an enological liquid that is still to be fermented (e.g. in order to obtain an antioxidant effect on a must), it may be preferable to have an inactive yeast available in which the breakage of the yeast molecules occurred in the most complete manner possible, in order to quickly execute the treatment before the enological liquid starts fermenting. In this case, the present process can advantageously provide for employing an apparatus for discontinuous cold pasteurization with lysis pressures of about 6000 bar. Advantageously, moreover, the present process can provide for multiple steps for pressurizing the active yeast within the pressurization chamber, in which such pressurization steps can all occur at the same lysis pressure or at increasing lysis pressures, for example a first step at lysis pressure of about 3000 bar, a second step at lysis pressure of about 4500 bar and a third step at lysis pressure of about 6000 bar.

In accordance with an important characteristic of the present invention, the active yeast is dough yeast or cream yeast or frozen yeast that was never dried, because experimentally it was confirmed that only with such yeast type is the lysis, obtained through the discontinuous or continuous cold pasteurization, is capable of providing the useful components contained in the cell membrane of the yeast, such as for example protein chains. The invention provides for a surprising synergistic effect due to the use of a yeast selected in the particular never-dried form and with water residue of at least 40%, and due to the use of a destructive process for the cell membrane of the same yeast, which is the cold pasteurization, in order to obtain a surprising effect of providing components of the yeast contained within the cell membrane. In addition, the presence of a considerable percentage of water residue within the selected yeast allows a uniform distribution of the lysis pressure on the cell membrane thereof, with consequent uniform breakage of the cell membranes of the yeast within the pressurization chamber. In particular, in the first embodiment, the yeast hermetically closed in its package will be subject to a particularly effective cell destructive action effect, due to the high pressure of the discontinuous cold pasteurization apparatus.

The particular process of cold pasteurization selected, i.e. of continuous or discontinuous type, unlike other pasteurization processes allows the nearly integral preservation of the protein chains contained in the original active yeast, which are then made available with the lysis of the cell membrane.

As mentioned above, the inactive yeast produced by the process, object of the present invention, can advantageously be employed in a method for treating an enological liquid, in which at least one step is provided for adding at least one dose of the inactive yeast, which is not intended to perform a fermentation action since it was inactive as previously indicated, i.e. with a cold pasteurization process that allows preserving intact all the characteristics of the products contained within the cell wall of the original active yeast, and such products are freed following the lysis of the yeast and are thus immediately available and ready to act.

Advantageously, the dose of inactive yeast to be added to the enological liquid will be substantially comprised between 5-50 g/hl for white wines and musts of white wines and between 10-80 g/hl for red wines and musts of red wines, depending on when the step occurs for adding the inactive yeast and on the desired wine-making purpose. Advantageously, the step of adding the inactive yeast can take place at any moment of the vinification process, from the pressing of the grapes to the maturation or refinement of the wine, also several months after the end of fermentation.

Advantageously, as mentioned above, the adding step can occur before the step of fermenting the enological liquid, i.e. on grapes or on musts, before these start to ferment. In this case, the inactive yeast is not intended to perform a fermentation and/or reproductive action, but rather it is intended to perform an antioxidant action of the must, or a nourishing action for the microbial population that will subsequently conduct the fermentation step.

In addition or alternatively, the adding step can advantageously occur after the fermentation step, i.e. after this step has been entirely completed. In this case, the inactive yeast is not intended to perform a fermentation action and/or reproductive action, but rather it is intended to perform an action of refinement or clarification of the wine.

Once inserted in the must and/or in the wine, the aforesaid dose of inactive yeast is responsible for various positive actions that determine a quality improvement of the wines. Such positive actions can regard: - the transfer of a colloidal fraction following lysis of the yeast cells, with the production of a wine that is sensorially provided with excellent characteristics of roundness, volume and persistence and with reduced irregular odors; - the stabilization of the wine against the precipitations of potassium bitartrate and proteins due to the transfer of mannoproteins by cells of the yeast, - the absorption of metals otherwise responsible for instability and for catalyst of oxidative reactions; - the absorption by the yeast cell walls of colored pigments, in particular of the white wines as well as of fermentation catabolites, antiparasite treatment residues, etc.; - the stabilization of the color in red wines; - the absorption of oxygen and consequent decrease of the wine oxidation; - the liberation of enzymes which contribute to positive evolutions of the wines; - the elimination of the reduction excesses.

Such positive effects can be seen in the graphs enclosed hereinbelow.

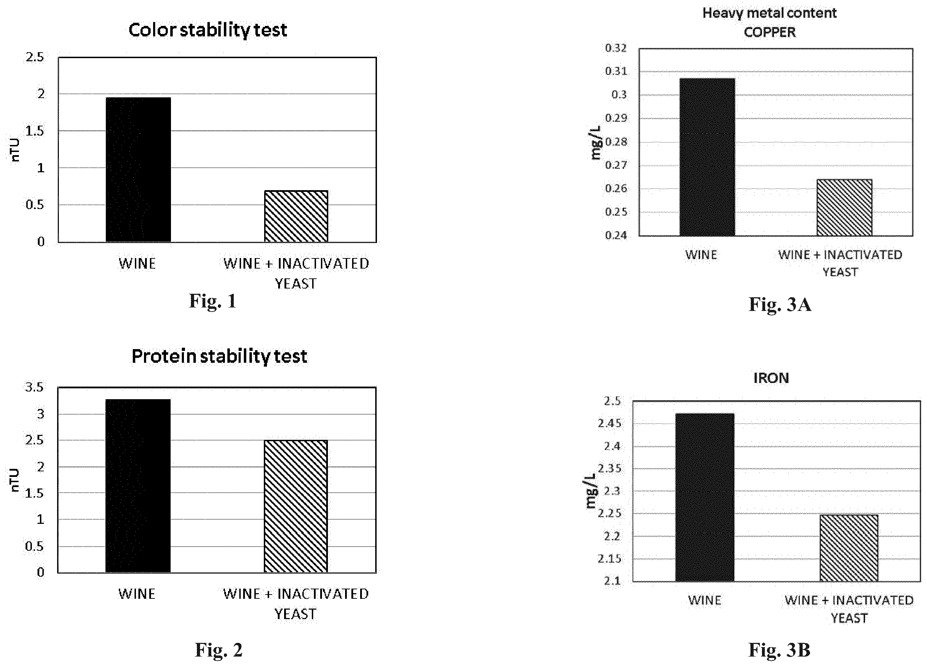

In particular, the graph of figure 1 allows comparing the stability of the color of a non-treated wine sample with respect to a sample of the same wine treated with the inactive yeast produced by means of the process, object of the present invention. More in detail, the wine analyzed is a Sangiovese, of which a first sample was not treated and was analyzed as is, and a second sample was treated with the addition of a dose of inactive yeast, produced by means of the process, object of the present invention, equal to 35 g/hl. The stability of the color was measured by evaluating, by means of turbidimeter, the variation of nephelometric turbidity units (NTU) of the two wine samples, after incubation at 4°C for 48 hours. As can be seen in the graph of figure 1, the wine sample treated with the inactive yeast has an NTU value clearly lower than the value of the untreated wine sample. In particular the value is less than half, passing from 1.94 NTU to 0.69 NTU.

The graph of figure 2 allows comparing the protein stability of a non-treated Chardonnay and of the same wine treated with 35 g/hl of inactive yeast. The protein stability was measured by means of a hot test, which provides for reading, by means of turbidimeter, the variation of nephelometric units (NTU) in the sample that took place from before a step of incubating the sample at 80° C for 45 minutes to after such step; this is followed by a step of cooling the sample at ambient temperature (about 20° C). As can be seen from the graph of figure 2, also with regard to the protein stability, the wine sample treated with the inactive yeast produced by means of the process, object of the present invention, has an NTU value lower than the value of the untreated wine sample. In particular the value of NTU has fallen from 3.25 to 2.5 NTU.

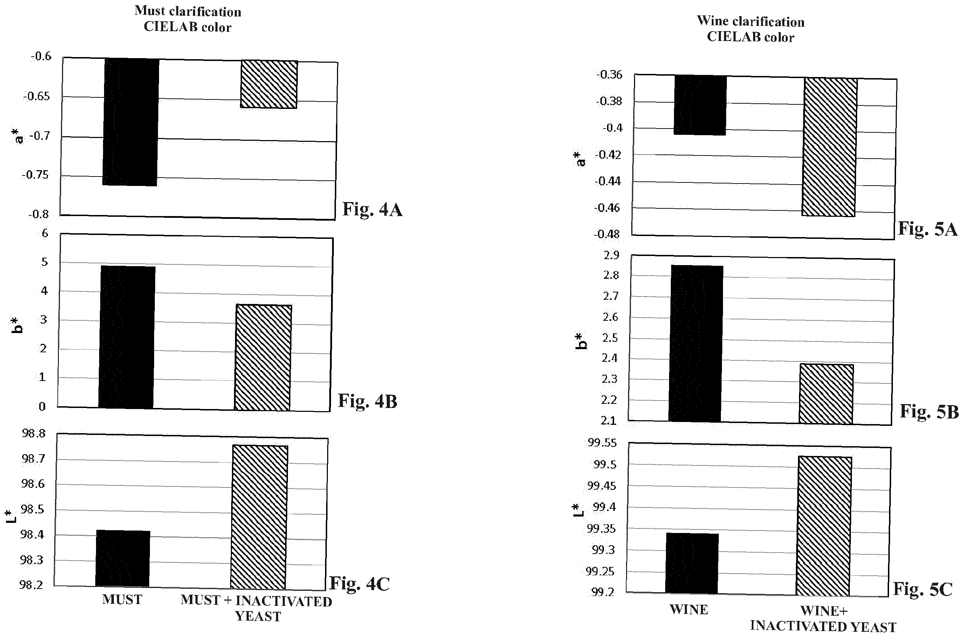

The two graphs of figure 3 allow comparing the quantity of heavy metals contained in the non-treated wine sample and treated wine sample analyzed in the graph of figure 2. In particular, the graph of figure 3A indicates the quantity of copper and the graph of figure 3B indicates the quantity of iron contained in the non-treated wine sample and in the treated wine sample, and such quantities were measured in mg of metal per liter of wine. From the comparison between the values measured in the non-treated wine sample and in the treated wine sample, it can be observed that the treatment with inactive yeast produced by means of the present process allows reducing the quantity of heavy metals contained in wine. In particular, the quantity of copper falls from 0.307 mg/L to 0.264 mg/L, while the quantity of iron falls from 2.471 mg/L to 2.246 mg/L. The graphs of figures 4 and 5 allow evaluating the clarification effect completed by a dose of inactive yeast produced with the present process, which was added respectively to a must sample and to a wine sample. More in detail, the graphs of figure 4 represent the color of a Prosecco must sample before and after the addition of a dose of inactive yeast equal to 35 g/hl. In particular, the color of the must was analyzed by means of CIELAB analysis, which expresses the color by means of three values: "a*" red quantity, "b*" yellow quantity and "L*" luminance (expressed in percentage, where 0% indicates the black color and 100% indicates the white color). The values of a*, b* and L* obtained are respectively reported in the graph of figures 4A, 4B and 4C. From the comparison between the values measured on the non-treated must and on the treated must, one can easily observe the clarification effect carried out by the inactive yeast. Indeed, the value of L* is increased from 98.42% to 98.76%.

Analogously, the graphs of figure 5 represent the color of a sample of a Prosecco base before and after the addition of a dose of inactive yeast equal to 35 g/hl. Also in this case the color was measured by means of CIELAB analysis and the values of a*, b* and L* obtained are respectively reported in the graph of figures 5A, 5B and 5C. Also for the wine, from the comparison between the values measured on non-treated sample and on the sample treated with the inactive yeast, one can observe the clarification effect carried out by the inactive yeast itself. Indeed, the value of L* is increased from 99.34 % to 99.53 %.

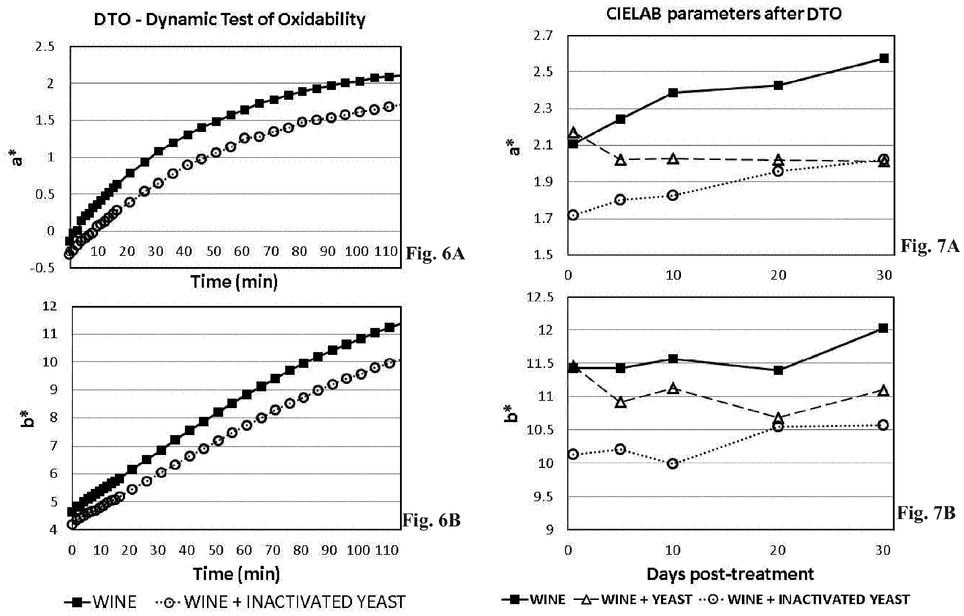

The graphs of figure 6 allow observing the protective effect against oxidations completed by a dose of inactive yeast produced with the present process, which was added to a wine sample. The protective effect was once again measured by means of CIELAB analysis, i.e. by evaluating the evolution of the color of a non-treated wine sample and of a treated wine sample, which were subjected to oxidative conditions (i.e. subjected to a temperature of 60° C and to the presence of H₂O₂). The obtained values of a* and b* are respectively reported in the graph of figures 6A and 6B. More in detail, the analyzed wine was a Manzoni Bianco, to which, in the treated sample, a dose of inactive yeast was added equal to 35 g/hl. In addition, the analysis of the color was repeated over time, at regular intervals, in order to evaluate the kinetics of oxidation of the wine during the two test hours in oxidative conditions. From the comparison between the red values (a*) and yellow values (b*) measured on the non-treated wine sample and on the treated wine sample with the deactivated yeast, one can observe that the treated sample has a more stable color over time. Indeed, both the value of a* and that of b* of the treated wine sample increase more slowly with respect to the same values of the non-treated wine sample.

The graphs of figure 7 allow observing the reduction of the refinement times of the wine due to the treatment of a wine sample by means of a dose of active yeast of known type, with respect to a wine sample treated with a dose of inactive yeast produced with the present process. Also in this case, the reduction of the refinement times was evaluated by means of CIELAB analysis, i.e. by measuring at a distance of 12 hours, 5 days, 10 days, 20 days and 30 days after treatment the parameters a* and b* of a non-treated wine sample, of a wine sample treated with active yeast and of a wine sample treated with inactive yeast, and such samples have been previously subjected to oxidative conditions for two hours of time (i.e. subjected to a temperature of 60° C and to the presence of H₂O₂). The values of a* and b* obtained are reported respectively in the graph of figures 7A and 7B. More in detail, the wine analyzed was the same Manzoni Bianco analyzed in the graphs of figure 6, to which, in the sample treated with active yeast, a dose of active yeast equal to 100 g/hl was added, while in the sample treated with inactive yeast, a dose of inactive yeast equal to 35 g/hl was added. In addition, the analysis of the color was executed after two hours of test in oxidative conditions and it was repeated over time in order to evaluate the progression of the oxidation of the wine in the days following the treatment. From the comparison between the red values (a*) and yellow values (b*) measured on the non-treated wine sample, on the wine sample treated with active yeast and on the wine sample treated with deactivated yeast, it can be observed that the sample treated with inactive yeast is that which shows lower values of a* and b* already 12 hours after the treatment, an indication of an immediate protective effect carried out by the inactive yeast, object of the present invention, against oxidations. Otherwise, the protective function of the active yeast achieves the maximum of its potential only 20-30 days after treatment, and leads to values of a* and b* that are lower than those of the non-treated wine and proximal to those of the wine treated with inactive yeast.

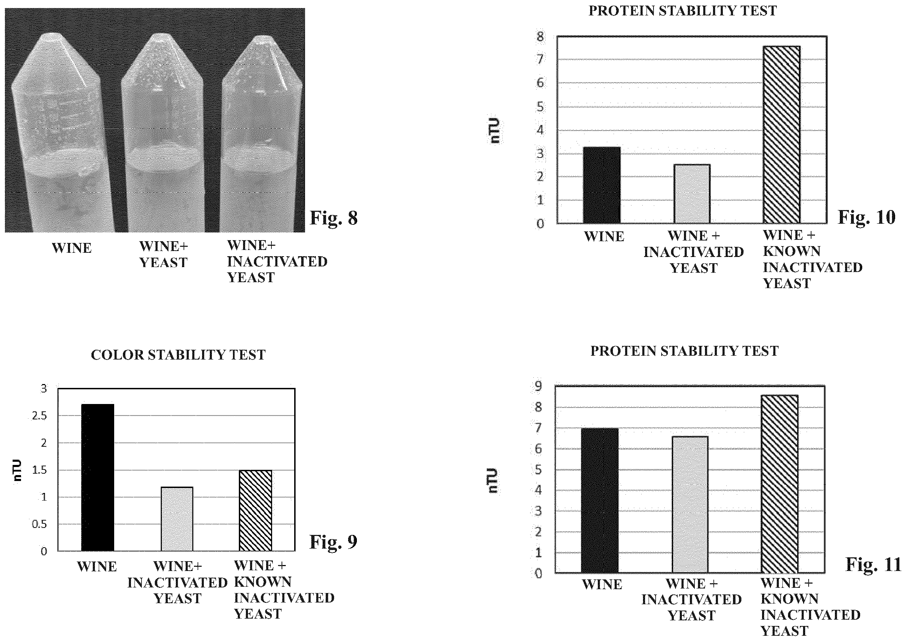

Figure 8 allows comparing the tartaric stability of a non-treated wine sample, with respect to the tartaric stability of a sample of the same wine treated with an active yeast of known type and with respect to the tartaric stability of a sample of the same wine treated with an inactive yeast produced with the present process. More in detail, the wine analyzed is a white wine of Custoza type, to which, in the sample treated with active yeast, a dose of active yeast equal to 100 g/hl was added, while in the sample treated with inactive yeast, a dose of inactive yeast equal to 30 g/hl was added. The aforesaid three samples were left to settle in the three containers visible in the photograph of figure 8, with the tips of the containers directed downward, for a sedimentation time of six days at the temperature of -4° C. Subsequently, the containers were overturned and arranged with the tips upward, as illustrated in the photograph of figure 8; in this manner, it was possible to more clearly observe the quantity of sediments left on the bottom of the containers (i.e. on the tips). From the comparison between the three samples it can be observed that the non-treated wine sample is highly unstable, indeed it has the greatest concentration of precipitate, and one observes the tip of the first container on the left which is nearly completely covered with sediment. However, the two containers containing the treated samples have a lower quantity of precipitate. In particular, from the comparison between the sample treated with active yeast and the sample treated with inactive yeast (respectively the second and the third sample from the left), it is possible to observe that the inactive yeast has a stabilization function greater than that of the active yeast, since the sample treated with inactive yeast is that which has the lowest concentration of precipitate.

Figures 9 to 27A-B allow comparing the action produced by the inactive yeast obtained with the present process with respect to an inactive yeast of known type, which was obtained starting from a dry yeast and was deactivated by means of thermal treatments. In particular, the same inactive yeast of known type was employed in all the analyses reported in the graphs of figures 9 to 27A-B.

In additions, figures 9 to 27A-B allow comparing the action produced by the two inactive yeasts with respect to a non-treated wine sample.

In particular, the graph of figure 9 allows comparing the stability of the color of a non-treated Merlot wine sample (2018 vintage) with respect to a sample of the same wine treated with the inactive yeast produced by means of the process, object of the present invention and with respect to a sample of the same wine treated with the inactive yeast of known type, which was obtained starting from a dry yeast and was deactivated by means of thermal treatments. More in detail, a first sample of the analyzed wine was not treated and was analyzed as is, a second sample was treated with the addition of a dose equal to 10 g/hl of inactive yeast produced by means of the process, object of the present invention; and a third sample was treated with the addition of a same dose of inactive yeast of known type. The stability of the color was measured by evaluating, by means of turbidimeter, the variation of nephelometric turbidity units (NTU) of the three wine samples, after incubation at 4°C for 48 hours. As can be seen from the graph of figure 9, the wine sample treated with the inactive yeast, object of the present invention, has a value of NTU clearly lower than the value of the wine sample as is and also lower than the wine sample treated with inactive yeast of known type. In particular the wine as is has a value of NTU equal to 2.71 NTU, the treated wine sample with the inactive yeast, object of the present invention, has a value of NTU equal to 1.17 NTU and the treated wine sample with the inactive yeast of known type has a value of NTU equal to 1.5 NTU.

The graph of figure 10 allows comparing the protein stability of a non-treated Garganega wine, with respect to the same wine treated with 10 g/hl of inactive yeast, object of the present invention, and with respect to the same wine treated with 10 g/hl of inactive yeast of known type. The protein stability was measured by means of a hot test, in which the variation of NTU that took place in the sample was measured from before to after an incubation step of the sample at 80° C for 45 minutes, followed by a step of cooling the sample at ambient temperature (about 20° C). As can be seen from the graph of figure 10, also with regard to the protein stability, the treated wine sample with the inactive yeast produced by means of the process, object of the present invention, has a value of NTU lower than the value of the wine sample as is and lower than the wine sample treated with the inactive yeast of known type, which instead worsens the protein stability of the wine. In particular the measured value of NTU is equal to 3.25 NTU for the wine as is, 2.5 NTU for the wine treated with the inactive yeast, object of the present invention, and is equal to 7.56 NTU for the wine treated with the inactive yeast of known type.

Similar to the preceding graph, also the graph of figure 11 allows comparing the protein stability of a sample of a non-treated Prosecco base, with respect to the same sample treated with 10 g/hl of inactive yeast, object of the present invention, and with respect to the same sample treated with 10 g/hl of inactive yeast of known type. Also in this case the protein stability was measured by means of hot test and results similar to the preceding graph were found. In particular a value of NTU was measured equal to 6.94 NTU for the sample as is, 6.55 NTU for the sample treated with the inactive yeast, object of the present invention, and equal to 8.56 NTU for the sample treated with the inactive yeast of known type.

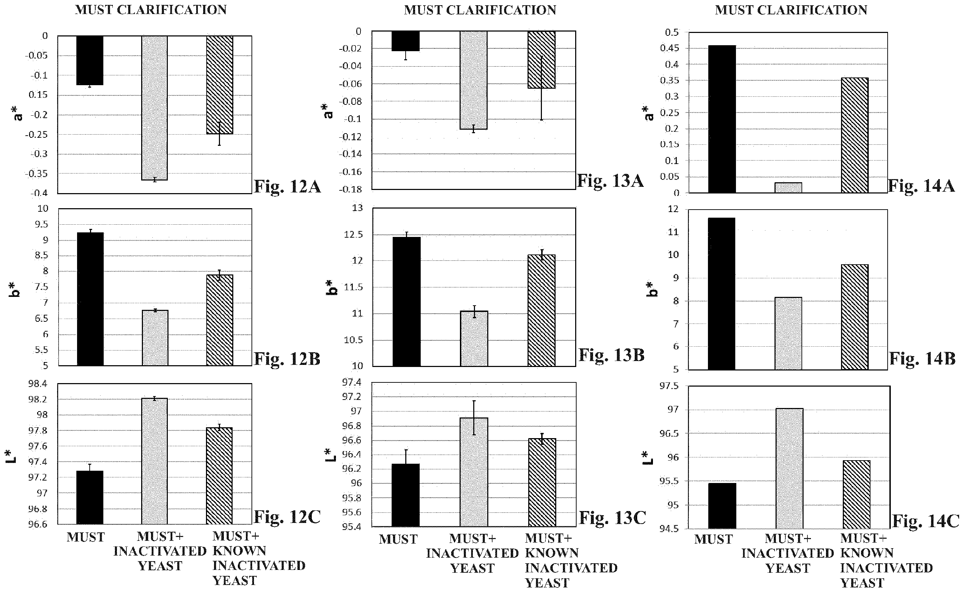

The graphs of figures 12, 13 and 14 allow comparing the clarification effect completed by a dose (10 g/hl) of inactive yeast produced with the present process, with respect to a same dose of inactive yeast of known type, which were added to three different must samples. In particular, the must of figure 12 is a Garganega must, the must of figure 13 is a Chardonnay must and the must of figure 14 is a Sauvignon blanc must.

More in detail, the graphs of figure 12 represent the color of a must sample of Garganega as is, after the addition of a dose of inactive yeast, object of the present invention, and after the addition of a same dose of inactive yeast of known type. The color was measured by means of CIELAB analysis and the values of a*, b* and L* obtained are respectively reported in the graph of figures 12A, 12B and 12C. From the comparison between the values measured on the three samples, one can easily note the clarification effect carried out by the present inactive yeast. In particular, the sample treated with the present inactive yeast has a value of L* (luminance) clearly greater than that of the other two samples, i.e. such must sample appears clearer than the other samples, and has clearly lower values of a* (red) and b* (yellow).

Analogously, the graphs of figure 13 represent the color of a must sample of Chardonnay as is, after the addition of a dose (10 g/hl) of inactive yeast, object of the present invention, and after the addition of a dose (10 g/hl) of inactive yeast of known type. Also in this case the color was measured by means of CIELAB analysis and the values of a*, b* and L* obtained are respectively reported in the graph of figures 13A, 13B and 13C. Also from these graphs, one can observe the clarification effect carried out by the present inactive yeast. In particular, the sample treated with the present inactive yeast has a value of L* clearly greater than that of the other two samples, and has clearly lower values of a* and b*.

Analogously, the graphs of figure 14 represent the color of a must sample of Sauvignon blanc as is, after the addition of a dose (10 g/hl) of inactive yeast, object of the present invention, and after the addition of a dose (10 g/hl) of inactive yeast of known type. Also in this case the color was measured by means of CIELAB analysis and the values of a*, b* and L* obtained are respectively reported in the graph of figures 14A, 14B and 14C. Also from these graphs, one can observe the clarification effect carried out by the present inactive yeast. In particular, the sample treated with the present inactive yeast has a value of L* clearly greater than that of the other two samples, and has clearly lower values of a* and b*.

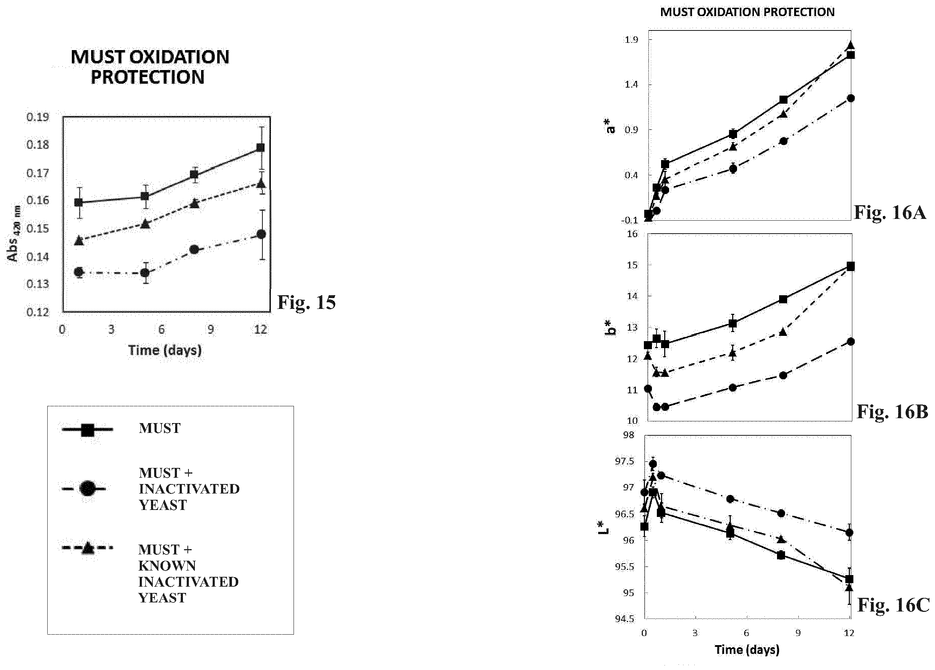

The graphs of figures 15 and 16 allow comparing the protective effect against oxidation completed by a dose (10 g/hl) of inactive yeast produced with the present process, with respect to a same dose of inactive yeast of known type, which were added to a must sample of Garganega. In addition, both the analyses of figures 15 and 16 were executed on samples placed at 30° C for twelve days. The graphs of figure 15 represent the absorbance at 420 nm of the different samples, i.e. they represent the fraction of incident light absorbed by each sample, which was read in reference to a light with wavelength centered at 420 nm, i.e. in reference to a wavelength centered on the yellow color and indicative of the presence of hydroxycinnamic acids, which are easily oxidizable phenolic compounds. In such analysis, therefore, high absorbance values correspond to high oxidation values of the sample. From the comparison between the three samples, it can be observed that the must sample treated with the present inactive yeast has lower oxidation, indeed its absorbance at 420 nm is lower than the other two samples. In particular, moreover, the value of absorbance of the sample treated with the present inactive yeast over time remains lower than the value of absorbance of the other two samples.

The graphs of figure 16 represent the color (measured by means of CIELAB analysis) of the three samples of the graph of figure 15, i.e. of the must sample of Garganega as is, after the addition of a dose of inactive yeast, object of the present invention, and after the addition of a dose of inactive yeast of known type. Also in this case, during the analysis, the samples were placed at 30° C for twelve days. The values of a*, b* and L* obtained are respectively reported in the graph of figures 16A, 16B and 16C. Also from the CIELAB analysis, one can observe that the level of a* (red) and b* (yellow) in the must sample treated with the present inactive yeast is lower than that of the other two samples and remains lower for the entire duration of measurements. In addition, also in this case it is possible to observe that the must sample treated with the present inactive yeast has values of L* greater than the other two samples, and also in this case the results remain the same over time.

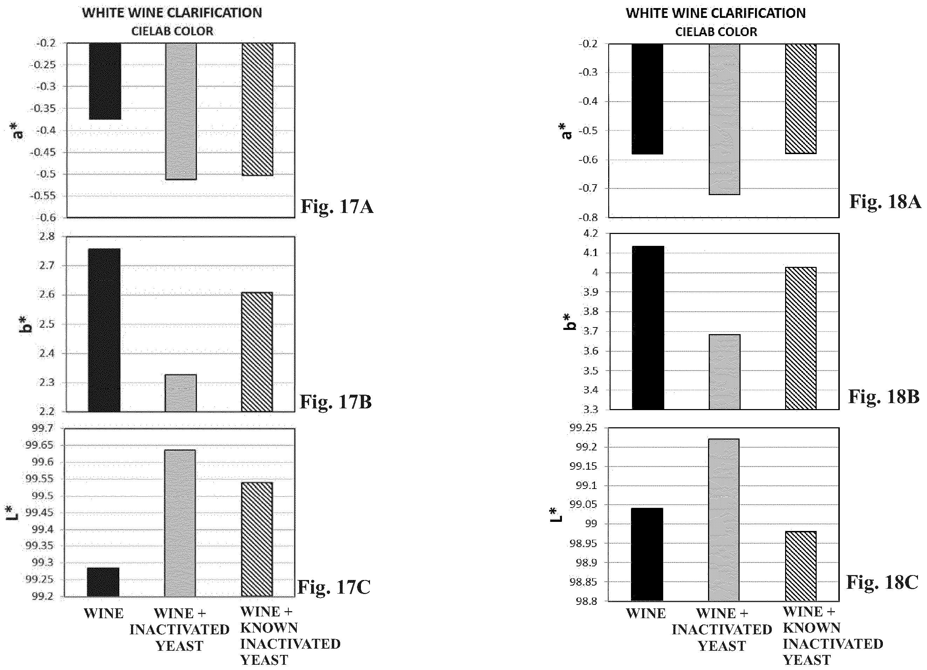

The graphs of figures 17 and 18 allow comparing the clarification effect completed by a dose (10 g/hl) of inactive yeast produced with the present process, with respect to a same dose of inactive yeast of known type, which were added to two different samples of white wine, and in particular to a Garganega wine sample (analyzed in figures 17A-C) and to a Sauvignon blanc sample (analyzed in figures 18A-C). Both graphs show the color (measured by means of CIELAB analysis) of the corresponding white wine sample as is, after the addition of a dose of inactive yeast object of the present invention and after the addition of a dose of inactive yeast of known type. In particular, the graphs of figures 17A, 17B and 17C respectively report the values of a*, b* and L* obtained on the samples of Garganega wine. From the comparison between the different measured values, one can observe the clarification effect carried out by the present inactive yeast since its value of L* is greater than that of the other two samples and its values of a* and b* are lower.

Analogously, the graphs of figures 18A, 18B and 18C respectively report the values of a*, b* and L* obtained on the samples of Sauvignon blanc wine. From the comparison between the different measured values, one can observe the clarification effect carried out by the present inactive yeast since its value of L* is greater than that of the other two samples and its values of a* and b* are lower.

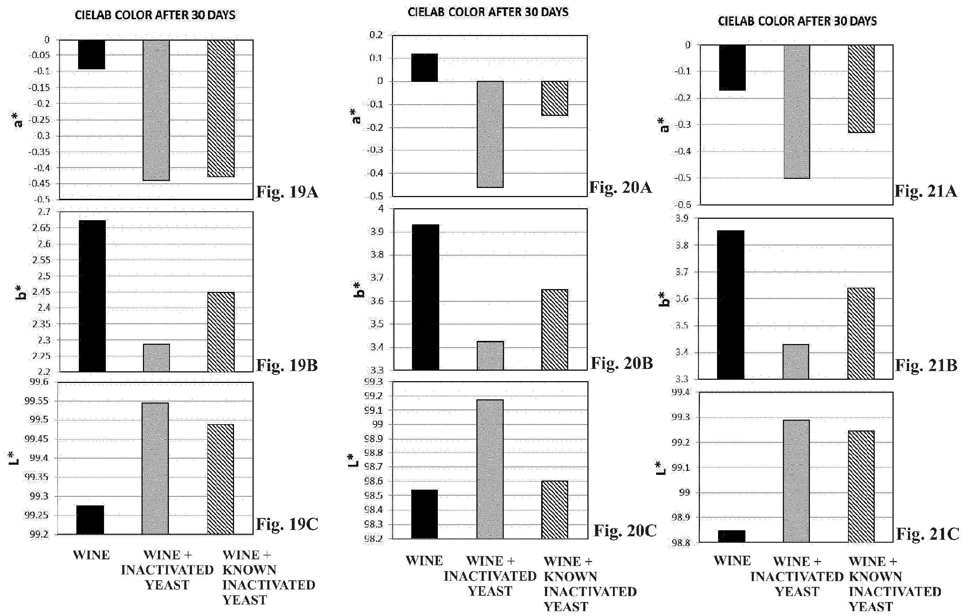

The graphs of figures 19, 20 and 21 allow analyzing the color (measured by means of CIELAB analysis) of three different samples of white wine after a thirty-day stop period. In particular, the three samples tested are a Garganega wine sample (analyzed in figures 19A-C), a sample of Chardonnay (analyzed in figures 20A-C) and a sample of Sauvignon blanc (analyzed in figures 21A-C). Each graph allows comparing the color of the corresponding wine sample as is, after the addition of a dose (10 g/hl) of inactive yeast produced with the present process and after the addition of a same dose of inactive yeast of known type. All the analyses were conducted thirty days after the treatment.

In particular, the graphs of figures 19A, 19B and 19C respectively report the values of a*, b* and L* obtained on the samples of Garganega wine. From the comparison between the different measured values, one can observe the effect of clarification and stabilization of the color carried out by the present inactive yeast. Indeed, its value of L* is greater than that of the other two samples and its values of a* and b* are lower. Analogously, the graphs of figures 20A, 20B and 20C respectively report the values of a*, b* and L* obtained on the samples of Chardonnay. From the comparison between the different measured values, one can observe the effect of clarification and stabilization of the color carried out by the present inactive yeast. Indeed, its value of L* is greater than that of the other two samples and its values of a* and b* are lower. Analogously, the graphs of figures 21A, 21B and 21C respectively report the values of a*, b* and L* obtained on the samples of Sauvignon blanc. Also in this case, from the comparison between the different measured values, one can observe the effect of clarification and stabilization of the color carried out by the present inactive yeast. Indeed, its value of L* is greater than that of the other two samples and its values of a* and b* are lower.

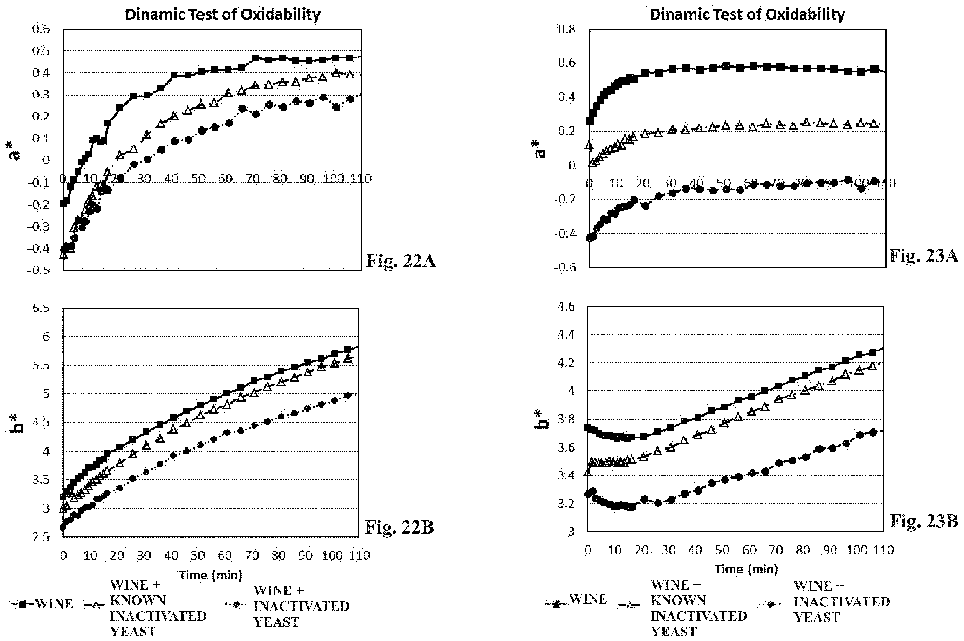

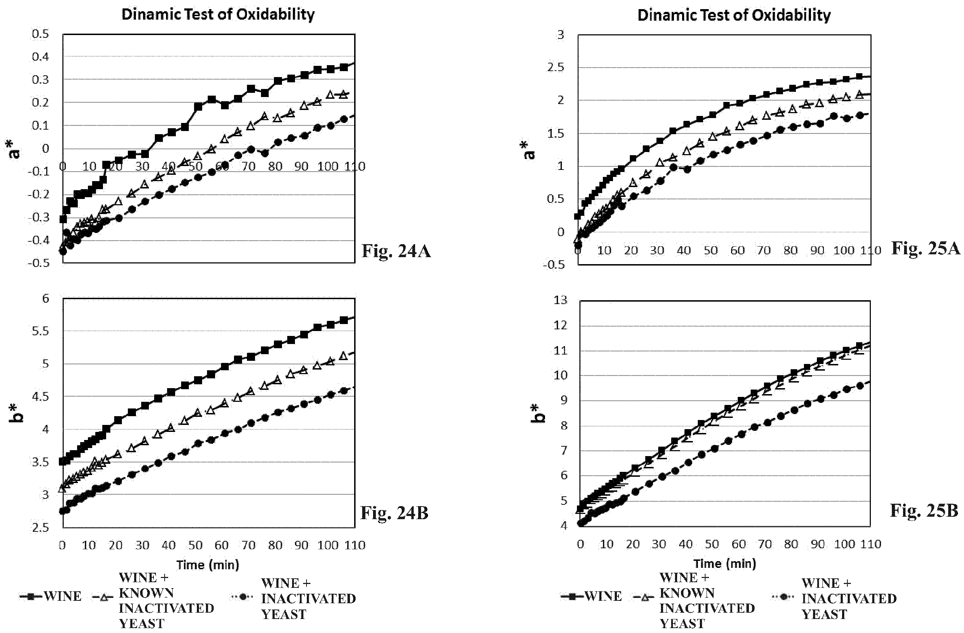

The graphs of figures 22 to 25 allow observing the protective effect against oxidation (measured by means of CIELAB analysis) of four different white wine samples, and in particular a Garganega wine sample (analyzed in figures 22A and 22B), a sample of Chardonnay (analyzed in figures 23A and 23B), a sample of Sauvignon blanc (analyzed in figures 24A and 24B), and a sample of a Prosecco base (analyzed in figures 25A and 25B). In particular, each graph allows comparing the color of the corresponding sample as is, after the addition of a dose (10 g/hl) of inactive yeast produced with the present process and after the addition of a same dose of inactive yeast of known type. All the analyses were conducted on wine samples subjected to oxidative conditions (i.e. subjected to a temperature of 60° C and to the presence of H₂O₂). In addition, the analysis of the color was repeated over time, at regular intervals, in order to evaluate the oxidation kinetics of the wine during the two test hours in oxidative conditions.

In particular, the graphs of figures 22A and 22B respectively report the values of a* (red) and b* (yellow) obtained on the samples of Garganega wine. From the comparison between the different measured values, one can observe that the sample treated with the present inactive yeast has a more stable color over time with respect to the other two samples, indeed both its value of a* and of b* increase more slowly with respect to the same values of the other two samples.

Analogous results are also obtained for the other three samples, as can be seen in the graphs 23A and 23B, 24A and 24B and 25A and 25B.

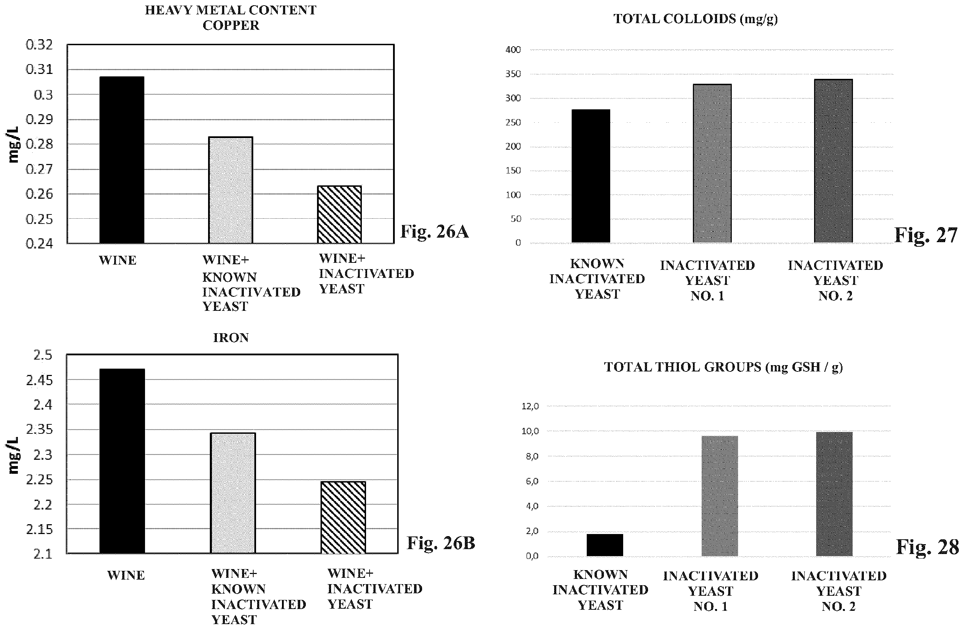

The two graphs of figure 26 allow comparing the quantity of heavy metals contained in a Merlot wine sample as is, in a sample of Merlot treated with a dose (10 g/hl) of inactive yeast, object of the present invention, and in a sample of Merlot treated with a same dose of inactive yeast of known type. In particular, the graph of figure 26A indicates the quantity of copper (measured in mg of copper per liter of wine) and the graph of figure 26B indicates the quantity of iron (measured in mg of iron per liter of wine) contained in the different samples. From the comparison between the measured values, one can observe that the sample treated with the present inactive yeast has a quantity of heavy metals lower than that of the wine as is and lower than that of the wine treated with inactive yeast of known type. In particular, the quantity of copper measured is equal to 0.307 for the wine as is, 0.283 for the wine treated with inactive yeast of known type and is equal to 0.263 for the wine treated with present inactive yeast. In addition, the quantity of iron measured is equal to 2.471 for the wine as is, 2.343 for the wine treated with inactive yeast of known type and is equal to 2.246 for the wine treated with present inactive yeast.

Figures 27 and 28 allow comparing the content of the inactive yeast of known type employed in the graphs of figures 9-26A-B (i.e. obtained starting from a dry yeast deactivated by means of thermal processes), with respect to two inactive yeasts obtained with the present process, of which one was subsequently dried by means of spray dry process (indicated in the enclosed graphs as "INACTIVATED YEAST NO. 1") and the other was subsequently dried by means of cold lyophilization process (indicated in the enclosed graphs as "INACTIVATED YEAST NO. 2"). In particular, the aforesaid two drying techniques are of *per se* known type and for this reason are not described in detail hereinbelow. The three inactive yeasts analyzed are all in dry form, since they are normally sold in such form.

The graph of figure 27 allows comparing the content of total colloids in the three different yeast samples. More in detail, with the expression "content of total colloids" it is intended the colloidal fraction present in the yeast sample, i.e. the macromolecules dispersed therein which are responsible for reacting with the colloidal fraction of the wine or of the must to which the yeast is added in order to obtain the stabilization effects analyzed in the preceding graphs. In particular, a higher value of colloids indicates greater respect of the original active yeast, since its protein chains are preserved in the form of longer protein chains. In addition, the presence of longer protein chains implies a greater stabilization effect of the wine or of the must to which such yeast is added, as is also confirmed by the results of the tests analyzed in the preceding figures. From the comparison between the values reported in figure 27, it can be seen that the inactive yeast of known type has a content of total colloids lower than that of the two inactive yeasts obtained with the present process. In particular the value of total colloids rises from 275 mg/g in the inactive yeast of known type to 330 mg/g in the present inactive yeast dried by means of spray-dry, up to 340 mg/g in the present inactive yeast dried by means of cold lyophilization.

The graph of figure 28 allows comparing the quantity of total thiol groups contained in the inactive yeast of known type with respect to the quantity of total thiol groups contained in the two inactive yeasts produced with the process, object of the present invention, and subsequently dried with a spray dry process (indicated in the enclosed graphs as "INACTIVATED YEAST NO. 1") and with a cold lyophilization process (indicated in the enclosed graphs as "INACTIVATED YEAST NO. 2"). Similar to the content of colloids, also for the thiol groups a higher value indicates greater respect of the original active yeast and allows obtaining a greater antioxidant effect on the wine or on the must to which such yeast is added, as confirmed also by the results of the tests analyzed in the preceding figures. From the comparison between the values reported in figure 28, it can be observed that the inactive yeast of known type has a content of thiol groups lower than that of the two inactive yeasts obtained with the present process. In particular the value of thiol groups is measured in mg of reduced glutathione for each gram of yeast (mgGHS/g) and is equal to 1.9 mgGHS/g in the inactive yeast of known type, to 9.8 mgGHS/g in the present inactive yeast dried by means of spray-dry and to 9.9 mgGHS/g in the present inactive yeast dried by means of cold lyophilization.

From all of the above-described results, it is clear that the addition of doses of inactive yeast, produced with the process, object of the present invention, to samples of wines and musts produces an improvement of the characteristics of the wine and of the must with respect to the same wine and must not treated and with respect to the same wine and must treated with an inactive yeast of known type.

In addition, from the graphs of figures 7A and 7B and from figure 8, it is clear that the treatment executed with the inactive yeast produced with the process, object of the present invention, improves the wine refinement times, as well as the tartaric stability of the wine, with respect to another type of yeast, in particular with respect to an active yeast of known type.

In addition, from the graphs of figures 9-26 it is clear that the addition of an inactive yeast obtained with the present process to musts and wines allows obtaining improved results of stabilization of starting must and wine with respect to the addition of an inactive yeast of known type.

In addition, from the graphs 27 and 28 it is clear that the yeast inactivated with the process, object of the present finding, more greatly preserves its original content with respect to the yeast inactivated with processes of known type. In addition, from the aforesaid graphs 27 and 28, it results that a subsequent drying by means of cold lyophilization is preferable with respect to a drying by means of spray-dry, even if the two techniques report rather similar values both regarding the content of colloidal groups and regarding the content of thiol groups.

The process thus conceived therefore attains the pre-established objects.

## Claims

1. Wine-making process which comprises:
- a step of arranging an enological product;
- a step of arranging an inactive yeast for wine-making use;
- a step of adding, to said enological product, said inactive yeast such that it does not perform a fermentation action in said process;
said method being **characterized in that** said step of arranging said inactive yeast comprises:
- providing an active yeast for wine-making use, never dried, with water residue of at least 40% by mass;
- inserting said active yeast in a pressurization chamber;
- inserting, within said pressurization chamber, a fluid at a lysis pressure higher than 1000 bar in order to subject said active yeast to said lysis pressure with consequent breakage of the molecular structure of said active yeast and obtainment of an inactive yeast lysate having at least one colloidal fraction and mannoproteins susceptible of reacting in wine or must;
- depressurizing said pressurization chamber;
- extracting said inactive yeast from said pressurization chamber.
said adding step being executed before or after a step of fermentation of said enological product.

2. Wine-making process according to claim 1, **characterized in that** said adding step is executed before a step of fermentation of said enological product; said enological product being composed of grapes or musts.

3. Wine-making process according to claim 1, **characterized in that** said adding step is executed after a step of fermentation of said enological product; said enological product being a wine.

4. Wine-making process according to any one of the claims 1 to 3, **characterized in that** said adding step provides for adding, to said enological product, a dose of said inactive yeast comprised between 5 and 80 g/hl of enological product.

5. Process for producing an inactive yeast for use in wine-making, which provides for:
- providing an active yeast for wine-making use, never dried, with water residue of at least 40% by mass;
- inserting said active yeast in a pressurization chamber;
- inserting, within said pressurization chamber, a fluid at a lysis pressure greater than 1000 bar in order to subject said active yeast to said lysis pressure with consequent breakage of the molecular structure of said active yeast and obtaining an inactive yeast lysate having at least one colloidal fraction and mannoproteins susceptible of reacting in wine or must;
- depressurizing said pressurization chamber;
- extracting said inactive yeast from said pressurization chamber.

6. Process for producing an inactive yeast according to claim 5, wherein said lysis pressure is comprised in an interval between 1000 and 6500 bar.

7. Process for producing an inactive yeast according to claim 6, wherein before the insertion of said active yeast in said pressurization chamber, a step is provided for inserting said active yeast in at least one package that is at least partly flexible.

8. Process for producing an inactive yeast according to claim 7, wherein following the insertion of said active yeast in said package and before the insertion of the package in said pressurization chamber, said package is sealed.

9. Process for producing an inactive yeast according to claim 6, wherein said fluid inserted in said pressurization chamber is water.

10. Process for producing an inactive yeast according to claim 5, wherein said lysis pressure is comprised in an interval between 1000 and 1500 bar.

11. Process for producing an inactive yeast according to claim 10, wherein said fluid inserted in said pressurization chamber is carbon dioxide.

12. Process for producing an inactive yeast according to any one of the preceding claims, wherein said active yeast is maintained at said lysis pressure for a time comprised between 30 and 600 seconds.

13. Process for producing an inactive yeast according to any one of the preceding claims, wherein said active yeast is dough yeast (COY).

14. Process for producing an inactive yeast according to any one of the claims 1 to 8, wherein said active yeast is cream yeast (CRY).

15. Process for producing an inactive yeast according to any one of the claims 1 to 8, wherein said active yeast is frozen yeast (AFY).

## Patentansprüche

1. Önologisches Verfahren, das Folgendes umfasst:
- einen Schritt der Vorbereitung eines önologischen Produkts;
- einen Schritt der Vorbereitung einer inaktiven Hefe zur Verwendung in der Önologie;
- einen Schritt der Zugabe der genannten inaktiven Hefe zu dem genannten önologischen Produkt, damit diese bei dem genannten Verfahren keine fermentierende Wirkung ausübt;
wobei das genannte Verfahren **dadurch gekennzeichnet ist, dass** der genannte Schritt der Vorbereitung der genannten inaktiven Hefe Folgendes umfasst:
- Bereitstellung einer aktiven Hefe zur Verwendung in der Önologie, die nie getrocknet wurde, mit einem Restwassergehalt von mindestens 40 % in der Masse;
- Hineingeben der genannten aktiven Hefe in eine Druckbeaufschlagungskammer;
- Hineingeben eines Fluids mit in die genannte Druckbeaufschlagungskammer bei einem Lysedruck von mehr als 1.000 bar, um die genannte aktive Hefe dem genannten Lysedruck auszusetzen, woraus sich der Zerfall der Molekularstruktur der genannten aktiven Hefe und der Erhalt einer lysierten inaktiven Hefe mit mindestens einer kolloidalen Fraktion und Mannoproteinen ergibt, die geeignet sind, im Wein oder Most zu reagieren;
- Druckabbau der Druckbeaufschlagungskammer;
- Entnahme der genannten inaktiven Hefe aus der genannten Druckbeaufschlagungskammer;
wobei der genannte Schritt der Zugabe vor oder nach einem Schritt der Fermentierung des genannten önologischen Produkts ausgeführt wird.

2. Önologisches Verfahren nach Anspruch Nr. 1, **dadurch gekennzeichnet, dass** der genannte Schritt der Zugabe vor einem Schritt der Fermentierung des genannten önologischen Produkts ausgeführt wird; wobei das genannte önologische Produkt aus Trauben oder Mosten besteht.

3. Önologisches Verfahren nach Anspruch Nr. 1, **dadurch gekennzeichnet, dass** der genannte Schritt der Zugabe nach einem Schritt der Fermentierung des genannten önologischen Produkts ausgeführt wird; wobei das genannte önologische Produkt ein Wein ist.

4. Önologisches Verfahren nach einem beliebigen der Ansprüche 1 bis 3, **dadurch gekennzeichnet, dass** der genannte Schritt der Zugabe vorsieht, dem genannten önologischen Produkt eine Dosis der genannten inaktiven Hefe zwischen 5 und 80 g/hl önologischem Produkt zuzusetzen.

5. Verfahren zur Herstellung einer inaktiven Hefe zur Verwendung in der Önologie, das Folgendes vorsieht:
- Bereitstellung einer aktiven Hefe zur Verwendung in der Önologie, die nie getrocknet wurde, mit einem Restwassergehalt von mindestens 40 % in der Masse;
- Hineingeben der genannten aktiven Hefe in eine Druckbeaufschlagungskammer;
- Hineingeben eines Fluids mit in die genannte Druckbeaufschlagungskammer bei einem Lysedruck von mehr als 1.000 bar, um die genannte aktive Hefe dem genannten Lysedruck auszusetzen, woraus sich der Zerfall der Molekularstruktur der genannten aktiven Hefe und der Erhalt einer lysierten inaktiven Hefe mit mindestens einer kolloidalen Fraktion und Mannoproteinen ergibt, die geeignet sind, im Wein oder Most zu reagieren;
- Druckabbau der Druckbeaufschlagungskammer;
- Entnahme der genannten inaktiven Hefe aus der genannten Druckbeaufschlagungskammer.

6. Verfahren zur Herstellung einer inaktiven Hefe nach Anspruch 5, bei dem der genannte Lysedruck in einem Bereich zwischen 1.000 und 6.500 bar liegt.

7. Verfahren zur Herstellung einer inaktiven Hefe nach Anspruch 6, bei dem vor dem Hineingeben der aktiven Hefe in die genannte Druckbeaufschlagungskammer ein Schritt des Unterbringens der genannten aktiven Hefe in mindestens einer mindestens teilweise flexiblen Packung vorgesehen ist.

8. Verfahren zur Herstellung einer inaktiven Hefe nach Anspruch 7, bei dem nach dem Unterbringen der genannten aktiven Hefe in der genannten Packung und vor dem Hineingeben der Packung in die genannte Druckbeaufschlagungskammer die genannte Packung versiegelt wird.

9. Verfahren zur Herstellung einer inaktiven Hefe nach Anspruch 6, bei dem das in die genannte Druckbeaufschlagungskammer gegebene genannte Fluid Wasser ist.

10. Verfahren zur Herstellung einer inaktiven Hefe nach Anspruch 5, bei dem der genannte Lysedruck in einem Bereich zwischen 1.000 und 1.500 bar liegt.

11. Verfahren zur Herstellung einer inaktiven Hefe nach Anspruch 10, bei dem das in die genannte Druckbeaufschlagungskammer gegebene genannte Fluid Kohlendioxid ist.

12. Verfahren zur Herstellung einer inaktiven Hefe nach einem beliebigen der vorangegangenen Ansprüche, bei dem die genannte aktive Hefe für einen Zeitraum zwischen 30 und 600 Sekunden bei dem genannten Lysedruck gehalten wird.

13. Verfahren zur Herstellung einer inaktiven Hefe nach einem beliebigen der vorangegangenen Ansprüche, bei dem die genannte aktive Hefe aus Presshefe besteht (COY).

14. Verfahren zur Herstellung einer inaktiven Hefe nach einem beliebigen der vorangegangenen Ansprüche 1 bis 8, bei dem die genannte aktive Hefe aus Cremehefe besteht (CRY).

15. Verfahren zur Herstellung einer inaktiven Hefe nach einem beliebigen der vorangegangenen Ansprüche 1 bis 8, bei dem die genannte aktive Hefe aus gefrorener Hefe besteht (AFY).

## Revendications

1. Procédé œnologique qui comprend :
- une phase de prédisposition d'un produit oenologique ;
- une phase de prédisposition d'une levure inactive pour un usage œnologique ;
- une phase d'addition audit produit œnologique de ladite levure inactive afin qu'elle n'exerce pas d'action de fermentation dans ledit procédé ;
ladite méthode étant **caractérisée en ce que** ladite phase de prédisposition de ladite levure inactive comprend :
- la mise à disposition d'une levure active pour un usage œnologique, jamais séchée, avec un résidu d'eau d'au moins 40% en masse ;
- l'insertion de ladite levure active dans une chambre de pressurisation ;
- l'insertion à l'intérieur de ladite chambre de pressurisation d'un fluide à une pression de lyse supérieure à 1000 bars pour soumettre ladite levure active à ladite pression de lyse avec une rupture consécutive de la structure moléculaire de ladite levure active et l'obtention d'une levure inactive lysée ayant au moins une fraction colloïdale et des mannoprotéines susceptibles de réagir dans le vin ou moût ;
- la dépressurisation de ladite chambre de pressurisation ;
- l'extraction de ladite levure inactive de ladite chambre de pressurisation ;
ladite phase d'addition étant exécutée avant ou après une phase de fermentation dudit produit œnologique.

2. Procédé œnologique selon la revendication 1, **caractérisé en ce que** ladite phase d'addition est exécutée avant une phase de fermentation dudit produit œnologique ; ledit produit œnologique étant composé de raisins ou de moûts.

3. Procédé œnologique selon la revendication 1, **caractérisé en ce que** ladite phase d'addition est exécutée après une phase de fermentation dudit produit œnologique ; ledit produit œnologique étant un vin.

4. Procédé œnologique selon l'une quelconque des revendications 1 à 3, **caractérisé en ce que** ladite phase d'addition prévoit d'additionner audit produit œnologique une dose de ladite levure inactive comprise entre 5 et 80 g/hl de produit œnologique.

5. Procédé pour la production d'une levure inactive pour un emploi en œnologie, qui prévoit :
- la mise à disposition d'une levure active pour un usage œnologique, jamais séchée, avec un résidu d'eau d'au moins 40% en masse ;
- l'insertion de ladite levure active dans une chambre de pressurisation ;
- l'insertion à l'intérieur de ladite chambre de pressurisation d'un fluide à une pression de lyse supérieure à 1000 bars pour soumettre ladite levure active à ladite pression de lyse avec une rupture consécutive de la structure moléculaire de ladite levure active et l'obtention d'une levure inactive lysée ayant au moins une fraction colloïdale et des mannoprotéines susceptibles de réagir dans le vin ou moût ;
- la dépressurisation de ladite chambre de pressurisation ;
- l'extraction de ladite levure inactive de la chambre de pressurisation.

6. Procédé pour la production d'une levure inactive selon la revendication 5, dans lequel ladite pression de lyse est comprise dans un intervalle entre 1000 et 6500 bars.

7. Procédé pour la production d'une levure inactive selon la revendication 6, dans lequel avant l'insertion de ladite levure active dans ladite chambre de pressurisation une phase d'insertion de ladite levure active est prévue dans au moins un emballage au moins en partie flexible.

8. Procédé pour la production d'une levure inactive selon la revendication 7, dans lequel après l'insertion de ladite levure active dans ledit emballage et avant l'insertion de l'emballage dans ladite chambre de pressurisation, ledit emballage est scellé.

9. Procédé pour la production d'une levure inactive selon la revendication 6, dans lequel ledit fluide inséré dans ladite chambre de pressurisation est de l'eau.

10. Procédé pour la production d'une levure inactive selon la revendication 5, dans lequel ladite pression de lyse est comprise dans un intervalle entre 1000 et 1500 bars.

11. Procédé pour la production d'une levure inactive selon la revendication 10, dans lequel ledit fluide inséré dans ladite chambre de pressurisation est du dioxyde de carbone.

12. Procédé pour la production d'une levure inactive selon l'une quelconque des revendications précédentes, dans lequel ladite levure active est maintenue à ladite pression de lyse pour un temps compris entre 30 et 600 secondes.

13. Procédé pour la production d'une levure inactive selon l'une quelconque des revendications précédentes, dans lequel ladite levure active est de la levure en pâte (COY).

14. Procédé pour la production d'une levure inactive selon l'une quelconque des revendications 1 à 8, dans lequel ladite levure active est de la levure en crème (CRY).

15. Procédé pour la production d'une levure inactive selon l'une quelconque des revendications 1 à 8, dans lequel ladite levure active est de la levure congelée (AFY).
